# EUROPÄISCHE PATENTSCHRIFT

(11) **EP 1 527 043 B1**
(45) Veröffentlichungstag und Bekanntmachung des Hinweises auf die Patenterteilung: **04.10.2006**
(21) Anmeldenummer: 03790873.8
(22) Anmeldetag: 07.08.2003
(51) Int. Cl.: C07C 213/02, C07C 213/10, C07B 49/00, C07C 217/74

(54) **VERFAHREN ZUR HERSTELLUNG VON 2- (DIMETHYLAMINO)METHYL -1-(3-METHOXYPHENYL)CYCLOHEXANOL**
METHOD FOR THE PRODUCTION OF 2- (DIMETHYLAMINO)METHYL|-1-(3-METHOXYPHENYL)CYCLOHEXANOL
PROCEDE POUR PRODUIRE DU 2- (DIMETHYLAMINO)METHYL|-1-(3-METHOXYPHENYL)CYCLOHEXANOL

(30) Priorität: 09.08.2002 DE 10236510
(43) Veröffentlichungstag der Anmeldung: 04.05.2005
(73) Patentinhaber: Grünenthal GmbH, 52078 Aachen (DE)
(72) Erfinder: FINKAM, Michael, 52066 Aachen (DE); AKTERIES, Bernhard, 52078 Aachen (DE)
(86) Internationale Anmeldenummer: PCT/EP2003/008746
(87) Internationale Veröffentlichungsnummer: WO 2004/020390

(56) Entgegenhaltungen:
- WO-A-99/03820
- WO-A-99/61405

## Beschreibung

Gegenstand der Erfindung ist ein Verfahren zur Herstellung von 2-[(Dimethylamino)methyl]-1-(3-methoxyphenyl)cyclohexanol mit einer hohen Stereoselektivität der trans Form aus den Enantiomeren (1R, 2R)- und (1S, 2S)-2-[(Dimethylamino)methyl]-1-(3-methoxyphenyl)cyclohexanol.

Tramadol, das aus historischen Gründen als "trans-Isomer" bezeichnet wird und die trans-Form aus den Enantiomeren (1R,2R)- (1S,2S)-2-[(Dimethylamino)methyl]-1-(3-methoxyphenyl)cyclohexanol umfaßt, ist analgetisch wirksam und wird daher als Analgetikum eingesetzt.

2-[(Dimethylamino)methyl]-1-(3-methoxyphenyl)cyclohexanol kann bekanntermaßen durch Grignard-Reaktion von 2-[(Dimethylamino)methyl]cyclohexanon und der Grignard-Verbindung von 3-Bromanisol hergestellt werden. Dabei werden sowohl die cis- als auch die trans-Form gebildet.

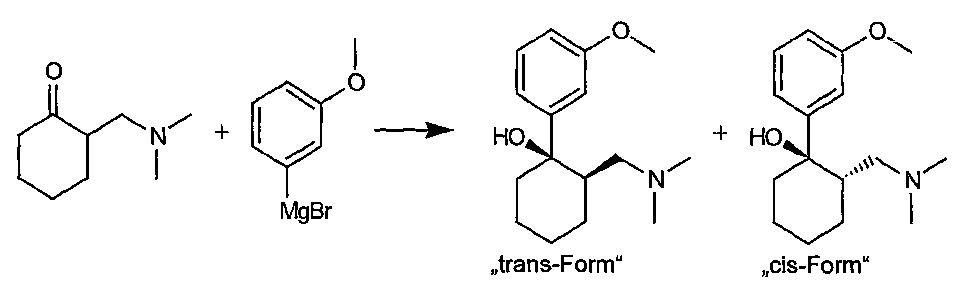

Die trans-Form, im folgenden als trans-Isomeres bezeichnet, beinhaltet das R, R und S, S Enantiomere mit den folgenden beiden Formeln:

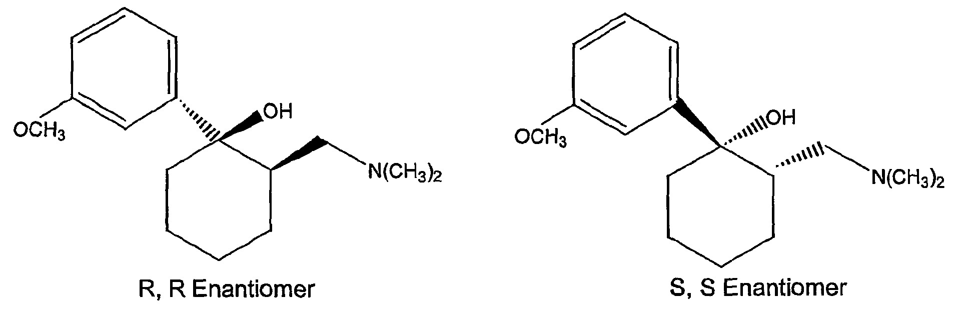

Die cis-Form, im folgenden als cis-Isomeres bezeichnet, beinhaltet das S, R und R, S isomere mit den beiden folgenden Formeln:

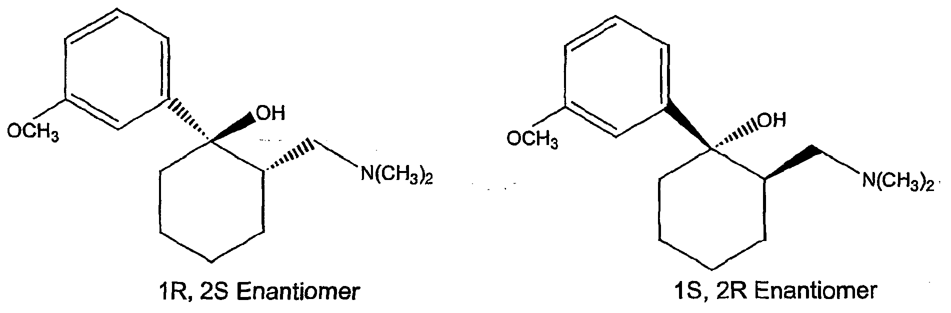

Bei der Herstellung von Tramadol mit Hilfe einer Grignard-Reaktion wird eine möglichst hohe Ausbeute des trans-Isomeren, der Tramadol Grignard Base angestrebt. Bekannt ist dabei bereits der Einfluß von Lösungsmitteln und Salzadditiven auf die Diastereoselektivität bei der Grignard-Reaktion.

Gemäß der Lehre von WO99/61405 wird bei der Herstellung von 2-[(Dimethylamino)methyl]-1-(3-methoxyphenyl)cyclohexanol eine Verschiebung des trans : cis-Isomeren-Verhältnisses von ca. 80:20 auf ca. 90:10 durch Einsatz von Aminen oder Ethern beschrieben. Die erzielte Ausbeute ist aber teilweise nur vergleichbar zu einer Reaktion ohne Zusätze, teilweise sogar deutlich schlechter.

Savelyev et.al. (international Conference on Natural Products and Physiologically Active Substances, Novosibirsk, 30.11.-6.12.1998, Poster) beschreiben ebenfalls eine Erhöhung der Diastereoselektivität bei der Herstellung von 2-[(Dimethylamino)methyl]-1-(3-methoxyphenyl)cyclohexanol durch Zusatz von Dioxanen, wobei sich aber die Ausbeute an cis- und trans-Isomer von 2-[(Dimethylamino)methyl]-1-(3-methoxyphenyl)cyclohexanol insgesamt verschlechtert.

Es stellte sich daher die Aufgabe, ein Verfahren zur Herstellung von 2-[(Dimethylamino)methyl]-1-(3-methoxyphenyl)cyclohexanol mit hoher Stereoselektivität für das trans Isomer und in hohen Ausbeuten zur Verfügung zu stellen.

Diese Aufgabe wird erfindungsgemäß durch ein verbessertes Verfahren zur Herstellung von 2-[(Dimethylamino)methyl]-1-(3-methoxyphenyl)cyclohexanol gelöst.

Gegenstand der Erfindung ist daher ein Verfahren zur Herstellung von 2-[(Dimethylamino)methyl]-1-(3-methoxyphenyl)cyclohexanol durch eine Grignard-Reaktion von 2-[(Dimethylamino)methyl]cyclohexanon mit der Grignard-Verbindung von 3-Bromanisol, Aufarbeitung des Reaktionsgemisches und ggf. Reinigung des entsprechenden Cyclohexanols, dadurch gekennzeichnet, daß man 2-[(Dimethylamino)methyl]cyclohexanon und die Grignard-Verbindung von 3-Bromanisol in einem geeigneten Lösungsmittel in Gegenwart eines anorganischen Lithiumsalzes und eines α, ω-Di-(C₁₋₃)-alkoxy-(C₁-C₃)alkans umgesetzt.

Vorzugsweise wird die Grignard-Verbindung von 3-Bromanisol in einem geeigneten Lösungsmittel vorgelegt, die Lösung mit einem anorganischen Lithiumsalz und einer entsprechenden Dialkoxy-Verbindung versetzt, ggf. nachgerührt und anschließend mit 2-[(Dimethylamino)methyl]cyclohexanon zur Umsetzung gebracht.

Die Lösung der Grignard-Verbindung von 3-Bromanisol wird vorzugsweise durch Umsetzung von 3-Bromanisol mit Magnesium in einem geeigneten Lösungsmittel besonders bevorzugt direkt vor der Umsetzung mit der Mannich Base hergestellt.

Das anorganische Lithiumsalz wird vorzugsweise in Mengen von 0.5-1 Äquivalent, bezogen auf 3-Bromanisol, eingesetzt. Vorzugsweise wird als anorganisches Lithiumsalz ein Lithiumhalogenid, besonders bevorzugt Lithiumchlorid verwendet.

Das Dialkoxyalkan wird vorzugsweise in Mengen von 20-120 Vol.-%, bezogen auf das vorstehend aufgeführte Lösungsmittel, zugesetzt. Vorzugsweise wird als Dialkoxyalkan 1,2-Dimethoxyethan verwendet.

Die Umsetzung von Magnesium und 3-Bromanisol zur Grignard-Verbindung erfolgt vorzugsweise bei einer Temperatur von 50-100°C.

Als Lösungsmittel für die Grignard-Verbindung wird vorzugsweise ein organisches Lösungsmittel, besonders bevorzugt Tetrahydrofuran verwendet.

Die Zugabe des anorganischen Lithiumsalzes, des Dialkoxyalkans und von 2-[(Dimethylamino)methyl]cyclohexanon zur Lösung der Grignard-Verbindung von 3-Bromanisol und die Umsetzung erfolgt vorzugsweise bei einer Temperatur von 0-60°C, besonders bevorzugt bei einer Temperatur von 15-35°C.

Zur Aufarbeitung des Reaktionsgemisches wird das Reaktionsgemisch vorzugsweise in gekühlte wäßrige Ammoniumchlorid-Lösung gegeben und nach der Phasentrennung die organische Phase vom Lösungsmittel befreit.

Überraschenderweise gelingt es durch die erfindungsgemäße Verfahrensweise sowohl die Ausbeute an der Grignard-Base als auch die Stereoselektivität zu Gunsten des gewünschten trans-Isomeren zu verbessern, d. h. bei einer hohen Ausbeute an der Grignard Base das trans : cis Isomeren Verhältnis deutlich zu steigern.

Das mit dem erfindungsgemäßen Verfahren erhaltene Gemisch der diastereomeren Grignard Basen kann in bekannter Weise zur Reinigung und insbesondere Trennung des trans Isomeren von dem cis Isomeren durch Umsetzung mit Salzsäure in das Hydrochlorid überführt und das Tramadol Hydrochlorid vorzugsweise aus Dioxan/Wasser umkristallisiert werden.

Selbstverständlich kann aus der erfindungsgemäß erhaltenen Grignard Base aus dem trans und cis Isomer das gewünschte trans Isomer nach jeder üblichen Trennungsmethode gewonnen werden.

Das erfindungsgemäß gewonnene Tramadol HCl kann als analgetischer Wirkstoff eingesetzt werden.

### Beispiele

### Beispiel 1

### A) Grignard-Reaktion

Die Reaktion wurde in einem 90 I-Kessel durchgeführt. 1,46 kg (60 mol) Magnesium-Späne wurden vorgelegt und bei 120°C ausgeheizt. Nach Zugabe von 18 l Tetrahydrofuran wurde auf eine Innentemperatur von 55°C aufgeheizt und zum Starten der Reaktion 0,5 kg 3-Bromanisol zugetropft. Nach dem Anspringen der Grignard-Reaktion wurden weitere 10,73 kg (insgesamt: 11,23 kg; 60 mol) 3-Bromanisol zugetropft und nachgerührt.
Dann wurde das Reaktionsprodukt auf 25°C abgekühlt, mit 1,91 kg (45 mol; 0,75eq bezogen auf 3-Bromanisol) Lithiumchlorid und 18 l Dimethoxyethan versetzt und 30 min nachgerührt.
Anschließend wurden 10,25 kg (66 mol)2-[(Dimethylamino)methyl]cyclohexanon über 2h zugetropft, wobei die Innentemperatur unter 30°C gehalten wurde. Nach der Zugabe wurde nachgerührt.
Das Reaktionsgemisch wurde langsam in gekühlte Ammoniumchlorid-Lösung aus 7,80 kg Ammoniumchlorid in 28,5 l Wasser gegeben und 0,5 h nachgerührt. Nach der Phasentrennung wurde die wäßrige Phase mehrmals mit Tetrahydrofuran extrahiert. Die organischen Phasen wurden vereinigt und über Magnesiumsulfat filtriert.
Dann wurde das Lösungsmittel im Vakuum entfernt, wobei man ein rot-braun gefärbtes Rohprodukt aus dem cis- und trans-Isomeren von 2-[(Dimethylamino)methyl]-1-(3-methoxyphenyl)cyclohexanol erhielt.

| | |
|---|---|
| Ausbeute: | 10,8 kg (68 % d. Th., HPLC) |
| Trans/Cis-Isomeren-Verhältnis: | 92 % : 8 % |

### B) Hydrochlorid-Fällung

1 kg des nach A) erhaltenen Rohproduktes aus dem cis- und trans-Isomeren wurde in Ether gelöst und mit trockenem Chlorwasserstoff versetzt. Das trans-Isomere des gebildeten Hydrochlorids von 2-[(Dimethylamino)methyl]-1-(3-methoxyphenyl)cyclohexanol wurde durch Umkristallisation aus Dioxan/ Wasser vom cis-Isomeren praktisch vollständig getrennt.

| | |
|---|---|
| Ausbeute: | 76 % d. Th. |

### Vergleichsbeispiel 1

### A) Grignard-Reaktion

Die Reaktion wurde in einem 90 I-Kessel durchgeführt. 1,46 kg (60 mol) Magnesium-Späne wurden vorgelegt und bei 120°C ausgeheizt. Nach Zugabe von 18 l Tetrahydrofuran wurde auf eine Innentemperatur von 55°C aufgeheizt und zum Starten der Reaktion 0,5 kg 3-Bromanisol zugetropft. Nach dem Anspringen der Grignard-Reaktion wurden weitere 10,73 kg (insgesamt: 11,23 kg; 60 mol) 3-Bromanisol zugetropft und nachgerührt.
Dann wurde das Reaktionsprodukt auf 25°C abgekühlt, mit 1,91 kg (45 mol; 0,75eq bezogen auf 3-Bromanisol) Lithiumchlorid und 14,7 l Tetrahydrofuran versetzt und 30 min nachgerührt.
Anschließend wurden 10,25 kg (66 mol)2-[(Dimethylamino)methyl]cyclohexanon über 2h zugetropft, wobei die Innentemperatur unter 30°C gehalten wurde. Nach vollständiger Zugabe wurde nachgerührt.
Das Reaktionsgemisch wurde langsam in gekühlte Ammoniumchlorid-Lösung aus 7,80 kg Ammoniumchlorid in 28,5 l Wasser gegeben und 0,5 h nachgerührt. Nach der Phasentrennung wurde die wäßrige Phase mehrmals mit Tetrahydrofuran extrahiert. Die organischen Phasen wurden vereinigt und über Magnesiumsulfat filtriert.
Dann wurde das Lösungsmittel im Vakuum entfernt, wobei man ein rot-braun gefärbtes Rohprodukt aus dem cis- und trans-Isomeren von 2-[(Dimethylamino)methyl]-1-(3-methoxyphenyl)cyclohexanol erhielt.

| | |
|---|---|
| Ausbeute: | 10,8 kg (68 % d. Th., HPLC) |
| Trans/Cis-Isomeren-Verhältnis: | 83 % : 17 % |

### B) Hydrochlorid-Fällung

1 kg des nach A) erhaltenen Rohproduktes aus dem cis- und trans-Isomeren wurde in Ether gelöst und mit trockenem Chlorwasserstoff versetzt. Das trans-Isomere des gebildeten Hydrochlorids von 2-[(Dimethylamino)methyl]-1-(3-methoxyphenyl)cyclohexanol wurde durch Umkristallisation aus Dioxan/ Wasser vom cis-Isomeren getrennt.

| | |
|---|---|
| Ausbeute: | 60 % d. Th. |

### Beispiel 2 und Vergleichsbeispiele 2 und 3

Es wurden weitere Versuche entsprechend Beispiel 1 bzw. Vergleichsbeispiel 1 mit und ohne Lithiumchlorid bzw. Dimethoxyethan, durchgeführt, wobei die Mengen der Reaktionskomponenten bzw. Additiven in Tabelle 1 zusammen mit einer Übersicht für Beispiel 1 und Vergleichsbeispiel 1 angegeben sind.

**Tabelle 1**

| | 3-Br-Anisol bzw. Magnesium | 2-[(Dimethylamino)methyl] cyclohexanon | THF | DME | LiCl | Ausbeute | Isomeren-Verhältnis | |
|---|---|---|---|---|---|---|---|---|
| | | | | | | | trans | cis |
| | mol | mol | ml | ml | mol | % | % | % |
| Beispiel 1 | 60 | 66 | 18000 | 18000 | 45 | 68 | 92 | 8 |
| Vergleichsbeispiel 1 | 60 | 66 | 32700 | 0 | 45 | 68 | 83 | 17 |
| Beispiel 2 | 0.45 | 0.50 | 135 | 60 | 0.45 | 70 | 92 | 8 |
| Vergleichsbeispiel 2 | 0.45 | 0.50 | 135 | 60 | 0 | 53 | 84 | 16 |
| Vergleichsbeispiel 3 | 0.45 | 0.50 | 135 | 0 | 0.45 | 70 | 87 | 13 |

| | | | | | | | | |
|---|---|---|---|---|---|---|---|---|
| THF = Tetrahydrofuran DME = 1,2 Dimethoxy ethan | | | | | | | | |

## Patentansprüche

1. Verfahren zur Herstellung von 2-[(Dimethylamino)methyl]-1 -(3-methoxyphenyl)cyclohexanol durch eine Grignard-Reaktion von 2-[(Dimethylamino)methyl]cyclohexanon mit der Grignard-Verbindung von 3-Bromanisol, Aufarbeitung und gegebenenfalls Reinigung des Reaktionsgemisches, **dadurch gekennzeichnet, daß** man 2-[(Dimethylamino)methyl]cyclohexanon und die Grignard-Verbindung von 3-Bromanisol in einem geeigneten Lösungsmittel in Gegenwart eines anorganischen Lithiumsalzes und eines α, ω-Di-(C₁₋₃)-alkoxy-(C₁-C₃)-alkans umsetzt.

2. Verfahren gemäß Anspruch 1, **dadurch gekennzeichnet, daß** man die Grignard-Verbindung von 3-Bromanisol in einem geeigneten Lösungsmittel vorlegt, die Lösung mit einem anorganischen Lithiumsalz und einem α, ω-Di-(C₁₋₃)-alkoxy-(C₁-C₃)-alkan versetzt, zu der Mischung 2-[(Dimethylamino)methyl]cyclohexanon hinzugibt und mit der Grignard-Verbindung reagieren läßt.

3. Verfahren gemäß Anspruch 1 oder 2, **dadurch gekennzeichnet, daß** die Lösung der Grignard-Verbindung von 3-Bromanisol durch Umsetzung von 3-Bromanisol und Magnesium in einem geeigneten Lösungsmittel direkt vor der Grignard-Reaktion hergestellt wurde.

4. Verfahren gemäß einem der Ansprüche 1-3, **dadurch gekennzeichnet, daß** das Lithiumsalz in Mengen von 0.5-1 Äquivalent, bezogen auf 3-Bromanisol, eingesetzt wird.

5. Verfahren gemäß einem der Ansprüche 1-4, **dadurch gekennzeichnet, daß** als Lithiumsalz Lithiumchlorid verwendet wird.

6. Verfahren gemäß einem der Ansprüche 1-5, **dadurch gekennzeichnet, daß** das Dialkoxy-α, ω-alkan in Mengen von 20-120 Vol.-%, bezogen auf das Lösungsmittel, zugesetzt wird.

7. Verfahren gemäß einem der Ansprüche 1-6, **dadurch gekennzeichnet, daß** als Dialkoxyalkan 1,2-Dimethoxyethan verwendet wird.

8. Verfahren gemäß einem der Ansprüche 1-7, **dadurch** gekennzeichet, daß als Lösungsmittel Tetrahydrofuran verwendet wird.

9. Verfahren gemäß einem der Ansprüche 1-8, **dadurch gekennzeichnet, daß** die Grignard-Reaktion bei einer Temperatur von 0-60°C erfolgt.

10. Verfahren gemäß Anspruche 9, **dadurch gekennzeichnet, daß** die Grignard-Reaktion bei einer Temperatur von 15-35°C, erfolgt.

11. Verfahren gemäß einem der Anspruche 1-8, **dadurch gekennzeichnet, daß** die Herstellung der Grignard-Verbindung bei einer Temperatur von 50-100°C, erfolgt.

12. Verfahren gemäß einem der Ansprüche 1-11, **dadurch gekennzeichnet, daß** zur Aufarbeitung das Reaktionsgemisch in gekühlte Ammoniumchlorid-Lösung gegeben und nach der Phasentrennung die organische Phase vom Lösungsmittel befreit wird.

13. Verfahren zur Herstellung von Tramadol HCl gemäß einem der Ansprüche 1-12, **dadurch gekennzeichnet, daß** das aufgearbeitete Reaktionsgemisch zur Abtrennung des trans Isomers (Tramadol) mit Salzsäure behandelt und das Hydrochlorid aus Dioxan/ Wasser umkristallisiert wird.

## Claims

1. Process for the preparation of 2-[(dimethylamino) methyl] -1- (3-methoxyphenyl)cyclohexanol by a Grignard reaction of 2-[(dimethylamino)methyl]cyclohexanone with the Grignard compound of 3-bromoanisole, working up of the reaction mixture and optionally purification of the reaction mixture, **characterized in that** 2-[(dimethylamino)methyl]cyclohexanone and the Grignard compound of 3-bromoanisole are reacted in a suitable solvent in the presence of an inorganic lithium salt and an α, ω-di- (C₁₋₃) -alkoxy- (C₁-C₃) alkane.

2. Process according to claim 1, **characterized in that** the Grignard compound of 3-bromoanisole is initially introduced into the reaction vessel in a suitable solvent, an inorganic lithium salt and an α, ω-di-(C₁₋₃)-alkoxy-(C₁-C₃)alkane is added to the solution, and 2-[(dimethylamino)methyl]cyclohexanone is added to the mixture and reacted with the Grignard compound.

3. Process according to claim 1 or 2, **characterized in that** the solution of the Grignard compound of 3-bromoanisole has been prepared directly before the Grignard reaction by reaction of 3-bromoanisole and magnesium in a suitable solvent.

4. Process according to one of claims 1-3, **characterized in that** the lithium salt is employed in amounts of 0.5-1 equivalent, based on the 3-bromoanisole.

5. Process according to one of claims 1-4, **characterized in that** lithium chloride is used as the lithium salt.

6. Process according to one of claims 1-5, **characterized in that** the dialkoxy-α,ω-alkane is added in amounts of 20-120 vol.%, based on the solvent.

7. Process according to one of claims 1-6, **characterized in that** 1,2-dimethoxyethane is used as the dialkoxyalkane.

8. Process according to one of claims 1-7, **characterized in that** tetrahydrofuran is used as the solvent.

9. Process according to one of claims 1-8, **characterized in that** the Grignard reaction is carried out at a temperature of 0-60 °C.

10. Process according to claim 9, **characterized in that** the Grignard reaction is carried out at a temperature of 15-35 °C.

11. Process according to one of claims 1-8, **characterized in that** the preparation of the Grignard compound is carried out at a temperature of 50-100 °C.

12. Process according to one of claims 1-11, **characterized in that** for working up, the reaction mixture is introduced into cooled ammonium chloride solution and, after separation of the phases, the organic phase is freed from the solvent.

13. Process for the preparation of tramadol HCl according to one of claims 1-12, **characterized in that**, to separate off the trans isomer (tramadol), the worked-up reaction mixture is treated with hydrochloric acid and the hydrochloride is recrystallized from dioxane/water.

## Revendications

1. Procédé de production de 2-[(diméthylamino)méthyl]-1-(3-méthoxyphényl)cyclohexanol par une réaction de Grignard entre la 2-[(diméthylamino)méthyl]cyclohexanone et le composé de Grignard du 3-bromanisole, traitement final et, le cas échéant, purification du mélange réactionnel, **caractérisé en ce qu'**on fait réagir de la 2-[(diméthyl-amino)méthyl]cyclohexanone et le composé de Grignard du 3-bromanisole dans un solvant convenable en présence d'un sel inorganique de lithium et d'un α, ω-di (alkoxy en C₁ à C₃) - (alcane en C₁ à C₃).

2. Procédé suivant la revendication 1, **caractérisé en ce qu'**on introduit d'abord le composé de Grignard du 3-bromanisole dans un solvant convenable, on ajoute un sel inorganique de lithium et un α, ω-di (alkoxy en C₁ à C₃)-(alcane en C₁ à C₃) à la solution, on ajoute au mélange de la 2-[(diméthylamino)méthyl]cyclohexanone et on fait réagir avec le composé de Grignard.

3. Procédé suivant la revendication 1 ou 2, **caractérisé en ce que** la solution de composé de Grignard du 3-bromanisole a été préparée par réaction de 3-bromanisole et de magnésium dans un solvant convenable immédiatement avant la réaction de Grignard.

4. Procédé suivant l'une des revendications 1 à 3, **caractérisé en ce que** le sel de lithium est utilisé en quantités de 0,5 à 1 équivalent par rapport au 3-bromanisole.

5. Procédé suivant l'une des revendications 1 à 4, **caractérisé en ce qu'**on utilise du chlorure de lithium comme sel de lithium.

6. Procédé suivant l'une des revendications 1 à 5, **caractérisé en ce que** le dialkoxy-α, ω-alcane est ajouté en quantités de 20 à 120 % en volume, par rapport au solvant.

7. Procédé suivant l'une des revendications 1 à 6, **caractérisé en ce qu'**on utilise comme dialkoxyalcane le 1,2-diméthoxyéthane.

8. Procédé suivant l'une des revendications 1 à 7, **caractérisé en ce qu'**on utilise comme solvant le tétrahydrofuranne.

9. Procédé suivant l'une des revendications 1 à 8, **caractérisé en ce que** la réaction de Grignard est conduite à une température de 0 à 60°C.

10. Procédé suivant la revendication 9, **caractérisé en ce que** la réaction de Grignard est conduite à une température de 15 à 35°C.

11. Procédé suivant l'une des revendications 1 à 8, **caractérisé en ce que** la préparation du composé de Grignard est effectuée à une température de 50 à 100°C.

12. Procédé suivant l'une des revendications 1 à 11, **caractérisé en ce que** pour le traitement final, le mélange réactionnel est versé dans une solution refroidie de chlorure d'ammonium et, après séparation des phases, la phase organique est débarrassée du solvant.

13. Procédé de production de chlorhydrate de tramadol suivant l'une des revendications 1 à 12, **caractérisé en ce que** le mélange réactionnel soumis au traitement final est traité à l'acide chlorhydrique en vue de la séparation de l'isomère trans (tramadol) et le chlorhydrate est recristallisé dans un mélange de dioxanne et d'eau.
